# EUROPEAN PATENT APPLICATION

(11) **EP 3 967 677 A1**
(43) Date of publication of application: **16.03.2022**
(21) Application number: 20802749.0
(22) Date of filing: 30.04.2020
(51) Int. Cl.: C07C 51/43, C07C 59/66

(54) **METHOD FOR PRODUCING BINAPHTHYL CARBOXYLIC ACID**

(30) Priority: 08.05.2019 JP 2019088292
(71) Applicant: Honshu Chemical Industry Co., Ltd., Tokyo 103-0027 (JP)
(72) Inventor: SAKUMA, Daichi, Wakayama-shi, Wakayama 641-0007 (JP); SUTO, Takeru, Wakayama-shi, Wakayama 641-0007 (JP); TSUCHIYA, Shinichi, Wakayama-shi, Wakayama 641-0007 (JP)
(74) Representative: Grünecker Patent- und Rechtsanwälte PartG mbB
(86) International application number: PCT/JP2020/018213
(87) International publication number: WO 2020/226114

(57) **Abstract**

An object is to provide a novel method for producing 2,2'-bis(carboxymethoxy)-1,1'-binaphthyl suitable as a resin raw material having excellent optical properties. As a solution, a method for producing 2,2'-bis(carboxymethoxy)-1,1'-binaphthyl, including a step (1) of performing crystallization, wherein the amount of water in a solution containing 2,2'-bis(carboxymethoxy)-1,1'-binaphthyl and one or more selected from linear or branched aliphatic ketones having 5 to 8 carbon atoms is in the range of 2.0 wt% or less and 0.01 wt% or more, is provided.

## Description

### Technical Field

The present invention relates to a method for producing a binaphthyl carboxylic acid. Particularly, the invention relates to a method for producing 2,2'-bis(carboxymethoxy)-1,1'-binaphthyl, the method using a crystallization solvent including a linear or branched aliphatic ketone having 5 to 8 carbon atoms and containing a specific amount of water.

### Background Art

Polyester resins and polyester carbonate resins produced using dicarboxylic acid components with binaphthalene skeletons as polymerization components have excellent optical properties such as high refractive indices and low birefringence and have high levels of heat resistance, and thus have recently been expected to be materials for optical members such as optical disks, transparent conductive substrates, and optical filters. In particular, resins produced using 2,2'-bis(carboxymethoxy)-1,1'-binaphthyl (hereinafter referred to as "compound A"), which has a chemical structure represented by a chemical formula below, as a polymerization component have been attracting attention for their particularly excellent optical properties (see, for example, PTLs 1 to 4).

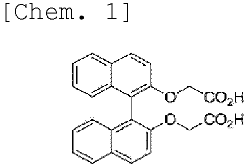

Known methods for producing compound A represented by the above formula include reacting 1,1'-binaphthalene-2,2'-diol with a halogenated acetic acid ester such as ethyl chloroacetate and hydrolyzing the resulting diester, as shown by a reaction formula below (see, for example, PTL 5). However, compound A obtained by this reaction is often used in the form of an acid chloride converted from an unpurified crude product with thionyl chloride, oxalyl chloride, or the like. Therefore, no studies or reports have ever been made on methods of purifying compound A.

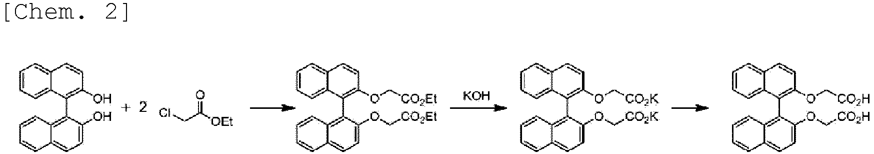

### Citation List

### Patent Literature

PTL 1: Japanese Unexamined Patent Application Publication No. 2001-072872
PTL 2: Japanese Unexamined Patent Application Publication No. 2018-002893
PTL 3: Japanese Unexamined Patent Application Publication No. 2018-002894
PTL 4: Japanese Unexamined Patent Application Publication No. 2018-002895
PTL 5: Japanese Unexamined Patent Application Publication No. 2008-024650

### Summary of Invention

### Technical Problem

The present invention has been made in view of the foregoing circumstances, and an object thereof is to provide a novel method for producing compound A suitable as a resin raw material having excellent optical properties.

### Solution to Problem

To achieve the above object, the present inventors have conducted intensive studies and discovered that when crystallization is performed using a specific solvent, the purification yield of highly pure compound A can be improved by controlling the amount of water in a crystallization solution to be in a specific range, thereby completing the present invention.

The present invention is as follows.
1. A method for producing 2,2'-bis(carboxymethoxy)-1,1'-binaphthyl, including a step (1) of performing crystallization, wherein the amount of water in a solution containing 2,2'-bis(carboxymethoxy)-1,1'-binaphthyl and one or more selected from linear or branched aliphatic ketones having 5 to 8 carbon atoms is in the range of 2.0 wt% or less and 0.01 wt% or more.
2. A method for producing 2,2'-bis(carboxymethoxy)-1,1'-binaphthyl, including steps (1) and (2):
   Step (1): the amount of water in a solution containing 2,2'-bis(carboxymethoxy)-1,1'-binaphthyl and one or more selected from linear or branched aliphatic ketones having 5 to 8 carbon atoms is adjusted to be in the range of 2.0 wt% or less and 0.01 wt% or more; and
   Step (2): 2,2'-bis(carboxymethoxy)-1,1'-binaphthyl is crystallized from the solution in the step (1).
3. The method for producing 2,2'-bis(carboxymethoxy)-1,1'-binaphthyl according to 1 or 2, wherein the 2,2'-bis(carboxymethoxy)-1,1'-binaphthyl in the step (1) is obtained by reaction between 1,1'-binaphthalene-2,2'-diol and a halogenated acetic acid or a halogenated acetic acid ester.

### Advantageous Effects of Invention

The present invention can provide highly pure compound A in much higher yield than conventional production methods. In particular, the present invention can improve the purification yield of compound A and thus is excellent as an industrial production method.

That is, the provision of the production method of the present invention is very useful in industrial use of resin raw materials and the like.

### Description of Embodiments

The present invention will be described below in detail.

Compound A of the present invention is a compound represented by a chemical formula below.

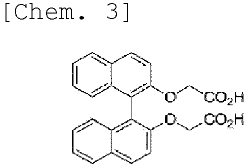

### <Regarding synthesis method>

Examples of methods for synthesizing compound A of the present invention include, but are not limited to, a known synthesis method (a) in which 1,1'-binaphthalene-2,2'-diol as a starting material is reacted with a halogenated acetic acid such as chloroacetic acid and a synthesis method (b) in which etherification for obtaining a diester by reaction of a halogenated acetic acid ester such as ethyl chloroacetate is performed and the diester is then hydrolyzed.

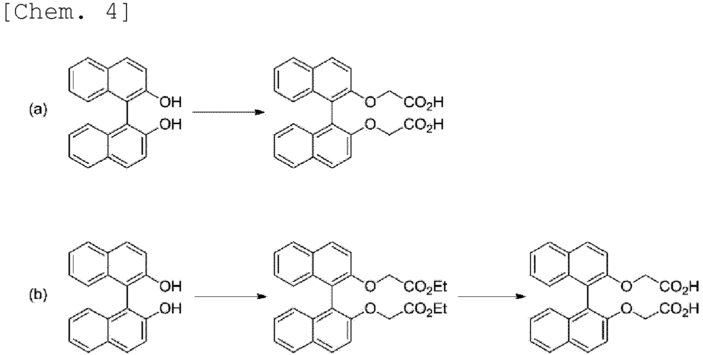

In the synthesis method (a), since the reaction is performed in the presence of a base such as an alkali metal carbonate such as lithium carbonate, sodium carbonate, or potassium carbonate or an alkali metal hydroxide such as lithium hydroxide, sodium hydroxide, or potassium hydroxide, compound A of interest is obtained as an alkali metal salt.

In the synthesis method (b), since the diester is hydrolyzed, compound A of interest is obtained as an alkali metal salt due to an alkali metal hydroxide such as lithium hydroxide, sodium hydroxide, or potassium hydroxide used in the hydrolysis reaction.

That is, the synthesis methods (a) and (b) both involve converting the alkali metal salt obtained to compound A by using an acid, and thus water washing treatment is necessary in order to remove inorganic matter produced during the conversion. The present inventors have newly discovered that if the water used in the water washing treatment is carried on to a subsequent step of crystallizing compound A, the purification yield of compound A obtained in the crystallization step is greatly reduced, thereby completing the present invention.

### <Regarding steps (1) and (2)>

The production method of the present invention is characterized in that crystallization is performed using at least one crystallization solvent selected from linear or branched aliphatic ketones having 5 to 8 carbon atoms.

Here, examples of usable linear or branched aliphatic ketones having 5 to 8 carbon atoms include diethyl ketone (which has 5 carbon atoms), methyl isobutyl ketone (which has 6 carbon atoms), methyl amyl ketone (which has 7 carbon atoms), and methyl hexyl ketone (which has 8 carbon atoms), among which methyl isobutyl ketone, methyl amyl ketone, and methyl hexyl ketone, which have low water solubility, are preferred.

As long as the advantageous effects of the present invention are not impaired, the crystallization solvent in the present invention may be combined with an organic solvent other than linear or branched aliphatic ketones having 5 to 8 carbon atoms, but is preferably composed only of a linear or branched aliphatic ketone having 5 to 8 carbon atoms.

Compound A used in the step (1) of the present invention may be in the form of, for example, crude crystals obtained by treating a reaction solution containing compound A, crystals obtained by recrystallizing the crude crystals, or a residual liquid left after distillation removal of solvent from a solution containing compound A. Compound A may be in amorphous form. Although compound A has two types of enantiomers, compound A used in the crystallization step of the present invention is preferably in racemic form, and the resulting crystals are also preferably in racemic form.

In the step (1) of the production method of the present invention, it is important to control the amount of water in a solution to be crystallized to be in the range of 2.0 wt% or less and 0.01 wt% or more. In particular, the upper limit is preferably 1.7 wt% or less, more preferably 1.0 wt% or less. The lower limit is preferably 0.03 wt% or more, more preferably 0.05 wt% or more.

The amount of water in the present invention means a water content measured by the Karl Fischer method.

The amount of linear or branched aliphatic ketone having 5 to 8 carbon atoms used in the step (1) is preferably 250 to 1000 parts by weight, more preferably 300 to 800 parts by weight, still more preferably 400 to 600 parts by weight, relative to 100 parts by weight of compound A. When all crystals are dissolved by increasing the temperature, the dissolution may be performed under normal pressure or increased pressure. When the amount of linear or branched aliphatic ketone having 5 to 8 carbon atoms is 450 parts by weight or less, the dissolution is preferably performed under increased pressure.

The crystallization may be performed by performing cooling after the amount of water in a solution obtained by dissolving compound A in a linear or branched aliphatic ketone having 5 to 8 carbon atoms is adjusted to be in the range of 2.0 wt% or less and 0.01 wt% or more. For example, precipitation of crystals by distilling the linear or branched aliphatic ketone out of the solution while adjusting the amount of water in the residual solution to be in the range of 2.0 wt% or less and 0.01 wt% or more and then performing cooling is convenient.

In the step (2) of the present invention, the temperature at which crystals are precipitated is preferably 90°C to 120°C, more preferably 95°C to 105°C. The time spent on solvent distillation is preferably 2 to 15 hours, more preferably 4 to 10 hours, still more preferably 6 to 8 hours.

The cooling rate during the crystallization by cooling and after the precipitation of crystals is preferably 5°C to 15°C per hour, more preferably 7°C to 12°C per hour. In precipitating crystals, seed crystals need not be used but are preferably used. Crystals obtained by a known production method or crystals precipitated without seed crystals may be used as seed crystals. The final cooling temperature is preferably 20°C to 60°C, more preferably 25°C to 35°C. After cooling to this temperature, precipitated crystals are separated by filtration.

### <Regarding drying step>

By drying the crystals obtained by the crystallization, the solvent used in the crystallization can be removed. The drying of the crystals obtained by the crystallization may be performed under normal pressure or reduced pressure. When the drying is performed on an industrial scale, it is preferably performed under reduced pressure because the solvent used in the crystallization can be removed more efficiently. The drying may be performed preferably under reduced pressure at 60°C to 120°C, more preferably under reduced pressure at 70°C to 110°C.

### EXAMPLES

The present invention will now be described more specifically with reference to Examples, but it should be noted that the present invention is not limited to these Examples.

### 1. Regarding method of analyzing amount of water

Measuring instrument: Karl Fischer MKS-520 (manufactured by Kyoto Electronics Manufacturing Co., Ltd.)

Titration was performed using 3 mg of AQUAMICRON Titrant SS as a titrant, and the amount of water was measured by volumetric titration.

### 2. Regarding method of analyzing purity

Measuring apparatus: high-performance liquid chromatography analyzer (manufactured by Shimadzu Corporation)
Pump: LC-20AD
Column oven: CTO-20A
Detector: SPD-20A
Column: HALO-C18
Oven temperature: 50°C
Flow rate: 0.7 ml/min
Mobile phase: (A) acetonitrile, (B) 0.1 vol% aqueous phosphoric acid water
Gradient conditions: (A) % by volume (time from start of analysis)
   30% (0 min) → 100% (12 min) → 100% (15 min)
Detection wavelength: 280 nm

After the measurement was performed under the above conditions, the purity (%) of target compounds obtained in Examples and Comparative Examples below was calculated using a liquid chromatography calibration curve of a target compound.

### <Synthesis Example 1>

### Synthesis of 2,2'-bis(carboxymethoxy)-1,1'-binaphthyl potassium salt

1,1'-Binaphthalene-2,2'-diol (1213 g), acetonitrile (3638 g), potassium carbonate (1346 g), and potassium iodide (121 g) were placed in a four-necked flask, heated to 70°C, and stirred at this temperature for one hour. After a mixed solution of ethyl chloroacetate (1460 g) and N-methylpyrrolidone (13 g) was prepared, the mixed solution was added dropwise while maintaining the temperature of a reaction solution at 70°C to 80°C. After stirring was performed for six hours, water (3032 g) was added, and the temperature was increased to 70°C, after which an aqueous layer was removed. Subsequently, a 35% aqueous potassium hydroxide solution (3392 g) was added dropwise while maintaining the temperature of a reaction solution at 70°C to 80°C. After two hours, the reaction solution was gradually cooled, and filtered at 25°C to obtain 2180 g of crystals of a potassium salt of 2,2'-bis(carboxymethoxy)-1,1'-binaphthyl.

### <Example 1>

Crystals obtained by drying the potassium salt obtained in Synthesis Example 1 (50.0 g), water (100 g), and methyl isobutyl ketone (265 g) were placed in a four-necked flask and heated to 80°C to be dissolved. Concentrated hydrochloric acid (30.0 g) was added dropwise while maintaining the temperature at 80°C to 85°C, and stirring was performed at this temperature for 30 minutes. After standing, an aqueous layer was extracted, and a water washing operation involving addition of water to the resulting oil layer, stirring, and removal of an aqueous layer by separation was performed multiple times until the pH of the aqueous layer reached 4. Subsequently, under normal pressure, water and methyl isobutyl ketone (147 g) were distilled out of the resulting oil layer. At 95°C midway through the distillation, seed crystals obtained by a production method known in the art were added, and precipitation of crystals was checked. The amount of water in the residual solution left after the distillation was 0.1%. The residual solution was cooled to 30°C at a cooling rate of 10°C per hour, filtered, and then dried to obtain 38.3 g of compound A in crystalline form. The yield was 91.0%, and the purity was 99.9%.

### <Example 2>

Crystals obtained by drying the potassium salt obtained in Synthesis Example 1 (50.0 g), water (100 g), and methyl isobutyl ketone (265 g) were placed in a four-necked flask and heated to 80°C to be dissolved. Concentrated hydrochloric acid (30.0 g) was added dropwise while maintaining the temperature at 80°C to 85°C, and stirring was performed at this temperature for 30 minutes. After standing, an aqueous layer was extracted, and a water washing operation involving addition of water to the resulting oil layer, stirring, and removal of water washing by separation was performed multiple times until the pH of the aqueous layer reached 4. Subsequently, under normal pressure, water and methyl isobutyl ketone (147 g) were distilled out of the resulting oil layer. At 95°C midway through the distillation, seed crystals obtained by a production method known in the art were added, and precipitation of crystals was checked. After this, water was added such that the amount of water in a residual solution would be 0.4%, and then the residual solution was cooled to 30°C at a cooling rate of 10°C per hour, filtered, and then dried to obtain 38.2 g of compound A in crystalline form. The yield was 90.9%, and the purity was 99.9%.

### <Example 3>

The same procedure as in Example 2 was performed except that the amount of water in the residual solution left after the distillation of water and methyl isobutyl ketone was adjusted to be 0.7%, thereby obtaining 38.2 g of compound A in crystalline form. The yield was 90.9%, and the purity was 99.9%.

### <Example 4>

The same procedure as in Example 2 was performed except that the amount of water in the residual solution left after the distillation of water and methyl isobutyl ketone was adjusted to be 1.0%, thereby obtaining 37.8 g of compound A in crystalline form. The yield was 90.1%, and the purity was 99.9%.

### <Example 5>

The same procedure as in Example 2 was performed except that the amount of water in the residual solution left after the distillation of water and methyl isobutyl ketone was adjusted to be 1.3%, thereby obtaining 36.8 g of compound A in crystalline form. The yield was 87.7%, and the purity was 99.9%.

### <Example 6>

The same procedure as in Example 2 was performed except that the amount of water in the residual solution left after the distillation of water and methyl isobutyl ketone was adjusted to be 1.7%, thereby obtaining 36.4 g of compound A in crystalline form. The yield was 86.6%, and the purity was 99.9%.

### <Comparative Example 1>

The same procedure as in Example 2 was performed except that the amount of water in the residual solution left after the distillation of water and methyl isobutyl ketone was adjusted to be 2.1%, thereby obtaining 33.4 g of compound A in crystalline form. The yield was 79.5%, and the purity was 99.9%.

### <Comparative Example 2>

The same procedure was performed except that methyl isobutyl ketone used in Example 1 was replaced with methyl ethyl ketone, while the amount of water in the solution was 0.1%, thereby obtaining 33.9 g of compound A in crystalline form. The yield was 80.7%, and the purity was 99.9%.

### <Comparative Example 3>

The same procedure as in Comparative Example 2 was performed except that the amount of water and methyl ethyl ketone distilled out was 210 g and the amount of water in the solution was changed to 3.2%, thereby obtaining 21.7 g of compound A in crystalline form. The yield was 51.7%, and the purity was 99.9%.

### <Comparative Example 4>

The same procedure was performed except that methyl isobutyl ketone used in Example 1 was replaced with cyclohexanone, while the amount of water in the solution was 0.1%, thereby obtaining 32.2 g of compound A in crystalline form. The yield was 76.7%, and the purity was 99.9%.

### <Comparative Example 5>

The same procedure as in Comparative Example 4 was performed except that the amount of water in the solution was changed to 1.5%, thereby obtaining 20.5 g of compound A in crystalline form. The yield was 48.8%, and the purity was 99.9%.

### <Comparative Example 6>

Crystals obtained by drying the potassium salt obtained in Synthesis Example 1 (50.0 g), water (100 g), and MIBK (265 g) were placed in a four-necked flask and heated to 80°C to be dissolved. Concentrated hydrochloric acid (30.0 g) was added dropwise while maintaining the temperature at 80°C to 85°C, and stirring was performed at this temperature for 30 minutes. After standing, an aqueous layer was extracted, and a water washing operation involving addition of water to the resulting oil layer, stirring, and removal of an aqueous layer by separation was performed multiple times until the pH of the aqueous layer reached 4. Without adjusting the amount of water in the oil layer, the oil layer was cooled to 30°C at a cooling rate of 10°C per hour, filtered, and then dried to obtain 24.9 g of 2,2'-bis(carboxymethoxy)-1,1'-binaphthyl in crystalline form. The yield was 59.2%, and the purity was 99.9%. The amount of water in the oil layer was 5.2%.

The test results of Examples 1 to 6 and Comparative Examples 1 to 6 are summarized in Table 1 below.

**[Table 1]**

| | | Solvent species | Amount of water (%) | Yield (%) |
|---|---|---|---|---|
| Example | 1 | methyl isobutyl ketone | 0.1 | 91.0 |
| | 2 | methyl isobutyl ketone | 0.4 | 90.9 |
| | 3 | methyl isobutyl ketone | 0.7 | 90.9 |
| | 4 | methyl isobutyl ketone | 1.0 | 90.1 |
| | 5 | methyl isobutyl ketone | 1.3 | 87.7 |
| | 6 | methyl isobutyl ketone | 1.7 | 86.6 |
| Comparative Example | 1 | methyl isobutyl ketone | 2.1 | 79.5 |
| | 2 | methyl ethyl ketone | 0.1 | 80.7 |
| | 3 | methyl ethyl ketone | 3.2 | 51.7 |
| | 4 | cyclohexanone | 0.1 | 76.6 |
| | 5 | cyclohexanone | 1.5 | 48.8 |
| | 6 | methyl isobutyl ketone | 5.2 | 59.2 |

As shown in Table 1, it was revealed that when methyl isobutyl ketone was used and the amount of water in the solution was in the range of 2.0 wt% or less and 0.01 wt% or more (Examples 1 to 6), the purification yield of compound A with a purity of 99.9% was 85% or more. It was confirmed that the efficiency with which highly pure compound A was obtained was greatly improved as compared to the production method in which the amount of water was not adjusted (Comparative Example 6).

Moreover, it was revealed from the results of Comparative Example 1 that controlling the amount of water in the solution to be in the range of 2.0 wt% or less and 0.01 wt% or more has critical significance.

In addition, it was also confirmed from the results of Comparative Examples 2 to 5 that this beneficial effect of improving the purification yield of highly pure compound A is produced by using a specific solvent.

It was revealed from the above that the effect produced by the production method of the present invention in which a linear or branched aliphatic ketone having 5 to 8 carbon atoms is used and the amount of water in a solution is in the range of 2.0 wt% or less and 0.01 wt% or more is an especially pronounced effect having critical significance.

## Claims

1. A method for producing 2,2'-bis(carboxymethoxy)-1,1'-binaphthyl, comprising a step (1) of performing crystallization, wherein an amount of water in a solution containing 2,2'-bis(carboxymethoxy)-1,1'-binaphthyl and one or more selected from linear or branched aliphatic ketones having 5 to 8 carbon atoms is in a range of 2.0 wt% or less and 0.01 wt% or more.

2. A method for producing 2,2'-bis(carboxymethoxy)-1,1'-binaphthyl, comprising steps (1) and (2):
Step (1): an amount of water in a solution containing 2,2'-bis(carboxymethoxy)-1,1'-binaphthyl and one or more selected from linear or branched aliphatic ketones having 5 to 8 carbon atoms is adjusted to be in a range of 2.0 wt% or less and 0.01 wt% or more; and
Step (2): 2,2'-bis(carboxymethoxy)-1,1'-binaphthyl is crystallized from the solution in the step (1).

3. The method for producing 2,2'-bis(carboxymethoxy)-1,1'-binaphthyl according to Claim 1 or 2, wherein the 2,2'-bis(carboxymethoxy)-1,1'-binaphthyl in the step (1) is obtained by reaction between 1,1'-binaphthalene-2,2'-diol and a halogenated acetic acid or a halogenated acetic acid ester.
